## Europäisches Patentamt

## European Patent Office

## Office européen des brevets

(19)

(11) Veröffentlichungsnummer: **0 015 433**
**A1**

(12) # EUROPÄISCHE PATENTANMELDUNG

(21) Anmeldenummer: 80100817.8

(22) Anmeldetag: 19.02.80

(51) Int. Cl.³: **A 61 B 17/00**, A 61 B 17/16,
**A 61 F 1/00, B 23 Q 3/12**

(30) Priorität: 10.03.79 DE 2909469

(43) Veröffentlichungstag der Anmeldung: 17.09.80
Patentblatt 80/19

(84) Benannte Vertragsstaaten: AT BE CH FR GB IT LU NL
SE

(71) Anmelder: Howmedica International, Inc.
Zweigniederlassung Kiel,
Professor-Küntscher-Strasse 1-5, D-2301 Schönkirchen
üb. Kiel (DE)

(72) Erfinder: Richter, Karl M.Dipl.-Ing., Haferkamp 14,
D-2304 Wendtorf (DE)
Erfinder: Harder, Hans Erich, Mecklenburger Str.32,
D-2301 Probsteierhagen (DE)
Erfinder: Behrens, Klaus, Am Sportplatz 8,
D-2351 Rickling (DE)

(74) Vertreter: Hauck, Hans, Dipl.-Ing. et al, Patentanwälte
Dipl.-Ing.H.Hauck, Dipl.-Phys.W.Schmitz,
Dipl.-Ing.E.Graalfs, Dipl.-Ing.W.Wehnert,
Dipl.-Phys.W.Carstens Dr.-Ing.W.Döring Neuer Wall 41,
D-2000 Hamburg 36 (DE)

(54) Spannvorrichtung für chirurgische Werkzeuge.

(57) Spannvorrichtung für chirurgische Werkzeuge, die ein auf einem Schaft (1) axial bewegbares Spannelement (4) aufweist, das in zwei axial beabstandeten Stellungen lösbar verriegelt ist. Das Spannelement (4) betätigt radial bewegliche Verriegelungselemente (5), welche in einer Raststellung des Spannelements (4) radial vorstehen und in Eingriff mit einem Werkzeug bringbar sind.

## Spannvorrichtung für chirurgische Werkzeuge

Die Erfindung bezieht sich auf eine Spannvorrichtung für chirurgische Werkzeuge, mit einem Schaft und einem auf dem Schaft angeordneten Spannorgan, das lösbar mit einem Werkzeug in Eingriff bringbar ist.

Spannvorrichtungen für chirurgische Werkzeuge, beispielsweise für Fräser, Bohrer oder dergleichen, müssen eine Reihe von Forderungen erfüllen. Die verwendeten Werkstoffe müssen aus korrosionsresistentem Material bestehen und leicht zu sterilisieren sein. Der Aufbau soll nach Möglichkeit einfach sein, um Herstellungs- und Materialkosten zu sparen. Gleichwohl soll die Einspannvorrichtung leicht zu handhaben und vielseitig anpaßbar sein und eine schnelle Wechselmöglichkeit beim Übergang auf ein anderes Werkzeug bieten.

0015433

Bekannte Einspannvorrichtungen erfüllen diese Forderungen nur teilweise. Daher liegt der Erfindung die Aufgabe zugrunde, eine einfach aufgebaute Einspannvorrichtung zu schaffen, welche leicht zu handhaben ist und für eine sichere Einspannung des Werkzeugs sorgt.

Diese Aufgabe wird erfindungsgemäß dadurch gelöst, daß ein flanschartiges Spannelement drehfest, jedoch axial beweglich zwischen zwei beabstandeten Raststellungen am Schaft angeordnet ist und mindestens ein Verriegelungselement radial gleitend im Spannelement geführt ist, von dem das radial innere Ende schwenkbar im Schaft gelagert ist, während das radial äußere Ende in der einen Raststellung zwecks Eingriffs in eine Ausnehmung eines Werkzeugs aus dem Spannelement vorsteht.

Durch einfaches axiales Verschieben kann bei der erfindungsgemäßen Spannvorrichtung von der Trennstellung in die Spannstellung und umgekehrt verstellt werden, indem das flanschartige Spannelement einfach von Hand erfaßt wird. Die Verriegelungselemente sind durch Formschluß gelagert und geführt und greifen formschlüssig in das entsprechend ausgebildete Werkzeug ein. Die erfindungsgemäße Spannvorrichtung zeichnet sich insbesondere durch ihre einfache Handhabung und ihre sichere Befestigung eines Werkzeugs aus. Sie kann sowohl mit einem Handgriff als auch mit einer chirurgischen Maschine angetrieben werden.

Zur Erzielung der axial beabstandeten Raststellungen sind verschiedene konstruktive Möglichkeiten denkbar und geeignet. Eine Ausgegestaltung der Erfindung sieht hierzu vor, daß zwischen Schaft und Spannelement eine oder mehrere radial elastisch gelagerte Kugeln angeordnet sind, die mit axial beabstandeten Ausnehmungen von Schaft oder Spannelement zusammenwirken. Die drehfeste Verbindung zwischen Spannelement und Schaft kann beispielsweise mit Hilfe einer Keilnutverbindung erfolgen. Vorzugsweise sind die Ausnehmungen im Schaft angeordnet und als Ringnuten ausgebildet, in die eine oder mehrere Kugeln des Spannelements einrasten.

Für die Lagerung der Kugeln sind wiederum verschiedene Möglichkeiten denkbar und geeignet. Eine Ausgestaltung der Erfindung sieht hierzu vor, daß die Kugeln jeweils in einer radialen Bohrung des Spannelements angeordnet sind und radial außen gegen ein elastisch nachgebendes, die Bohrung umgebendes Ringelement anliegen, das seinerseits in einer Ringausnehmung des Spannelements angeordnet ist und das Ringelement von einem von einem Federelement, vorzugsweise einer dünnen Hülse umgeben ist. In diesem Fall ist das Spannelement in diesem Bereich vorzugsweise ringzylindrisch geformt. Die Hülse kann geschlossen, jedoch auch axial geschlitzt sein.

Während der axialen Verstellung das Spannelements nehmen die Verriegelungselemente eine unterschiedliche Lage ein, da Spannelement und Schaft relativ zueinander axial bewegt werden. Es ist daher vorteilhaft, wenn gemäß einer weiteren Ausgestaltung der Erfindung die Verriegelungselemente Stifte sind, die in radialen Schlitzen des Spannelements angeordnet sind und die radial inneren Enden der Verriegelungsstifte mit einer Ausnehmung im Schaft zusammenwirken. Die Schwenkachse der Verriegelungsstifte liegt somit im Bereich der Ausnehmung im Schaft. Zur reibungsarmen Lagerung ist es darüber hinaus zweckmäßig, wenn gemäß einer weiteren Ausgestaltung der Erfindung die radial inneren Enden der Stifte kugelförmig geformt sind. Es ist zwar denkbar, den Schaft der Spannvorrichtung selbst so auszubilden, daß er mit den Verriegelungselementen zusammenwirkt. Eine vorteilhafte Ausgestaltung der Erfindung sieht hingegen vor, daß der mit einer Bohrung des Spannelements zusammenwirkende Endabschnitt des Schaftes von einem mit den Verriegelungselementen zusammenwirkenden Endelement gebildet wird. Das Endelement wird beispielsweise durch Verschraubung mit dem Schaft verbunden, so daß es entsprechend dem gewünschten Zusammenwirken mit den Verriegelungselementen ausgebildet sein kann. Darüber hinaus wird die Montage der Spannvorrichtung erleichtert.

Insbesondere in der Spannstellung muß gewährleistet sein, daß durch von außen aufgebrachte axiale Kraft keine Relativbewegung zwischen Spannelement und Schaft stattfindet, so daß die Raststellung verlassen wird. Daher sieht eine weitere Ausgestaltung der Erfindung vor, daß zum Einspannen des Werkzeugs Spannelement und Schaft aufeinander zu bewegt werden und der Schaft eine Schulter aufweist, die in der Spannstellung mit einem axialen Anschlag des Spannelements zusammenwirkt. Auf diese Weise wird die volle axiale Kraft über Schulter und Anschlag auf das Werkzeug übertragen, während die Verrastung zwischen Spannelement und Schaft lediglich die umgekehrte Verstellung in Richtung Trennstellung verhindern soll.

Entsprechend kann gemäß einer weiteren Ausgestaltung der Erfindung in der Trennstellung das Endelement mit einem axialen Anschlag des Spannelements zusammenwirken. Dadurch wird verhindert, daß Spannelement und Schaft unabsichtlich völlig voneinander getrennt werden.


Bei einer Bewegung von Spannelement und Schaft aufeinander zu wird der Lagerpunkt eines Verriegelungselements am Schaft vom Spannelement axial fortbewegt. Damit nun das Verriegelungselement eine entsprechende Schwenkbewegung ausführt, ist ein entsprechendes Widerlager am Spannelement vorzusehen. Dieses Widerlager kann in irgendeiner geeigneten Art und Weise am Spannelement ausgebildet sein. Eine Ausgestaltung der Erfindung sieht vor, daß die radialen Schlitze axial nach außen offen sind und die Stirnseite des Spannelements durch eine im Durchmesser kleinere Scheibe abgedeckt wird, gegen deren Außenumfang sich die Stifte bei der Verstellung von der Trenn- in die Spannstellung anlegen. Bei einer Verstellung in umgekehrter Richtung können sich die Stifte an einem

entgegengesetzten Widerlager des Spannelements abstützen.


Die Verriegelung des vom Spannelement aufgenommenen Werkzeugs erfolgt bei der Erfindung dadurch, daß die Verriegelungselemente sich mehr oder weniger radial nach außen erstrecken. Daher sind sie in der Trennstellung zur Schaftachse geneigt angeordnet und gemäß einer weiteren Ausgestaltung der Erfindung bei Verstellung in die Spannstellung geringfügig über ihre Totpunktlage, in der sie in einer radialen Ebene liegen, hinaus verstellbar. In der Spannstellung sind dann die Verriegelungsstifte wiederum mehr oder weniger zur Schaftachse geneigt, so daß die in das Werkzeug eingreifenden

0015433

Enden gleichzeitig eine kraftschlüssige Verspannung vornehmen
können.

Die erfindungsgemäße Einspannvorrichtung sorgt für eine feste
und sichere Einspannung eines Werkzeugs, ist leicht zu handhaben,
läßt eine schnelle Wechselmöglichkeit zu und ist vielseitig anpaßbar. Darüber hinaus ist sie einfach herstellbar und montierbar.

Ein Ausführungsbeispiel der Erfindung soll nachfolgend anhand von
Zeichnungen näher beschrieben werden.

Fig. 1   zeigt im Schnitt eine Spannvorrichtung nach der Erfindung

Fig. 2   zeigt einen Teil der Vorrichtung nach Fig. 1, jedoch in
         einer anderen Arbeitsstellung.

Fig. 3   zeigt eine Einzelheit der Vorrichtung nach Fig. 1.

Bevor auf die in den Zeichnungen dargestellten Einzelheiten näher
eingegangen wird, sei vorangestellt, daß jedes der beschriebenen
Merkmale für sich oder in Verbindung mit Merkmalen der Ansprüche
von erfindungswesentlicher Bedeutung ist.

Ein zylindrischer Schaft 1, der am rechten Ende beispielsweise
mit einer chirurgischen Maschine gekoppelt werden kann, ist im
mittleren Bereich von einem Handgriff 2 umgeben, der mittels einer
Schraube 3 axial fest, jedoch drehbar angeordnet ist. Das vordere

0015433

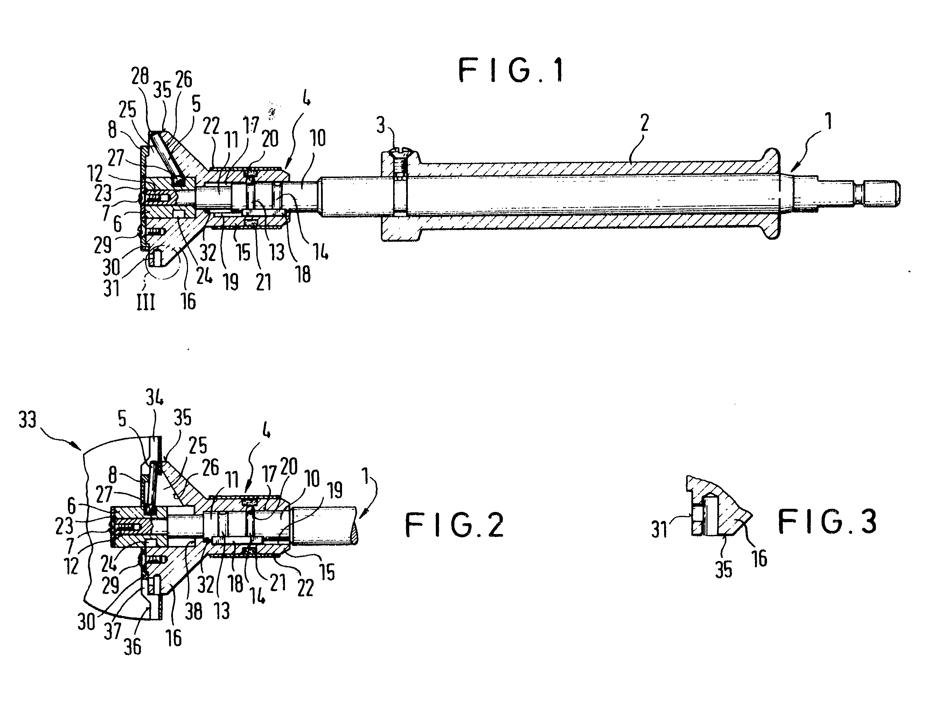

Ende des Schafts 1 besitzt drei abgestufte Abschnitte 10, 11 und
12 mit jeweils gegenüber dem vorhergehenden Abschnitt kleineren
Durchmesser, wie aus den Figuren 1 und 2 zu erkennen. Der Schaftabschnitt 10 besitzt axial beabstandet zwei Ringnuten 13, 14. Um
das vordere Ende des Schafts 1 ist ein allgemein mit 4 bezeichnetes Spannelement angeordnet, das einen hinteren zylinderringförmigen Abschnitt 15 und einen vorderen konischen Abschnitt 16 besitzt. Der Spannelementabschnitt 15 hat eine zentrische Bohrung
17, deren Durchmesser dem Durchmesser des Abschnitts 10 entspricht.
Mit Hilfe eines Keils 18 und einer Nut 19 ist das Spannelement 4
drehfest auf dem Schaft 1 angeordnet.

Im Spannelementabschnitt 15 sind mehrere Kugeln in radialen zur
Bohrung 17 hin offenen Löchern angeordnet, von denen eine bei
20 dargestellt ist. An der Außenseite besitzt der Abschnitt 15
eine Ringnut, in der ein Ring 21 aus einem elastisch nachgebendem
Material angeordnet ist. Auf dem Außenumfang des Abschnitts 15 ist
eine dünne metallische Buchse 22 angeordnet, so daß die Kugel 20
unter Überwindung einer Federkraft radial nach außen gedrückt
werden kann. Die Kugel 20 besitzt normalerweise entweder in der
Ringnut 13 oder der Ringnut 14 (letztere Stellung in Fig. 2 dargestellt).

Auf dem vorderen dünnen Abschnitt 12 des Schaftes 1 sitzt ein
Endstück 6, das mit Hilfe einer Schraube 7 und einer Scheibe 23
auf dem Schaft gesichert ist, in dem über die Scheibe 23 das
Endstück gegen die Schulter zwischen Abschnitt 11 und Abschnitt 12
des Schaftes 1 gepreßt wird. Das Endstück 6 besitzt am Außenumfang eine Ringnut 24.

Der Spannelementabschnitt 16 besitzt mehrere radiale nach außen und nach vorn offene Schlitze, von denen einer bei 25 zu sehen ist. Die Schlitze sind in der Längsschnittdarstellung trapezförmig gestaltet und haben dadurch eine schräg zur Schaftachse verlaufende Wand 26. Innerhalb der Schlitze 25 sitzen Verriegelungsstifte 5, die am radial inneren Ende eine Kugel 27 aufweisen. Die Kugel 27 ist in der Ringnut 24 des Endstücks 6 gelagert. In der in Fig. 1 gezeigten Stellung, der Trennstellung, liegt der Verriegelungsstift 5 annähernd an der Wand 26 an und erstreckt sich mit dem radial äußeren Ende zur radialen Öffnung 28 des Schlitzes 25, ragt jedoch nicht darüber hinaus.

Die vordere offene Seite der Schlitze 25 ist durch eine Scheibe 8 abgeschlossen, welche mit Hilfe mehrerer Schrauben, von denen eine bei 29 dargestellt ist, an der Frontseite des Spannelementenabschnitts 16 befestigt ist. Die Scheibe 8 besitzt einen axial verstärkten Flansch 30, der mit einer nicht bezeichneten Schulter des Spannelementenabschnitts 16 an dessen Stirnseite zusammenwirkt. An der Stirnseite ist zwecks Zentrierung eines Werkzeuges eine umlaufende Ringnut 31 eingeformt.

In der in Fig. 1 gezeigten Trennstellung nehmen die Verriegelungsstifte eine Schräglage ein, die etwa einen Winkel von 60° zur Schaftachse einschließt. Wird nun das Spannelement 4 von Hand erfaßt und nach rechts bewegt, rastet die Kugel 20 aus der Ringnut 13 heraus und rastet bei entsprechender fortgesetzter Verstellung schließlich in die Ringnut 14. Die Länge der Bohrung 17 im Abschnitt 15 ist nun so bemessen, daß nach Zurücklegen dieses Verstellweges die Schulter zwischen den Abschnitten 10 und 11 des Schaftes 1 gegen eine Schulter 32 der Bohrung 17 anliegt. Eine weitere Relativbewegung der genannten Teile aufeinanderzu ist damit begrenzt. Da das Endstück 6 relativ zum Schaft 1 stationär ist, bewegt sich bei der beschriebenen Verstellung die Kugel 27 der Verriegelungsstifte 5 vom Spannelement 4 nach außen fort, wobei sich der Verriegelungsstift gegen den Flansch 30 der Scheibe 8 anlegt, so daß er sich der gemeinsamen Radialebene für alle Verriegelungsstifte nähert. Wie Fig. 2 jedoch zeigt, in der die Spannstellung der gezeigten Spannvorrichtung dargestellt ist, wird diese Radialebene überschritten und der Spannstift wird geringfügig entgegengesetzt geneigt.

Vor dem beschriebenen Spannvorgang wird ein allgemein mit 33 bezeichnetes, nicht näher beschriebenes Werkzeug auf das Spannelement gesetzt. Das Werkzeug besitzt mindestens die gleiche Anzahl von radialen Öffnungen 34 wie Spannstifte 5 vorhanden sind. Eine nicht bezeichnete innenzylindrische Fläche des Werkzeugs 33 hat den gleichen Durchmesser wie eine zylindrische Außenfläche 35 des Abschnitts 16 des Spannelements 4, so daß beide Flächen axial übereinandergeschoben werden können. Eine radiale Fläche 36 am Werkzeug legt sich gegen die Stirnseite des Spannelements 16 an und begrenzt das Aufschieben des Werkzeugs auf das Spannelement 4. Ein axialer ringförmiger Steg 37 wirkt mit der Ringnut 31 zusammen, damit die radialen Bohrungen 34 des Werkzeugs 33 mit den radialen Öffnungen 28 der

0015433

Schlitze 25 zur Zusammenwirkung gebracht werden. Die Spannstifte 5 können dann bei der beschriebenen Bewegung in die Spannstellung in die radialen Bohrungen 34 des Werkzeugs 33 eingreifen und bei einer Verstellung über die Totpunktlage hinaus das Werkzeug außerdem verspannen.

Das Lösen des Werkzeugs 33 erfolgt durch Verstellung des Spannelements 4 von der in Fig. 2 gezeigten in die in Fig. 1 gezeigte Stellung.

0015433

<u>A n s p r ü c h e</u> :

1. Spannvorrichtung für chirurgische Werkzeuge, mit einem Schaft, einem Spannelement, das den Schaft hülsenartig umgebend gegenüber diesem axial bewegbar ist, mindestens einem zwischen Schaft und Spannelement wirkenden, begrenzt radial beweglichen ersten Verriegelungselement, das einer axialen Relativbewegung von Schaft und Spannelement einen Widerstand entgegensetzt, mindestens einem annähernd radial angeordneten, am Schaft angreifenden zweiten Verriegelungselement zur Übertragung des Drehmoments vom Schaft auf das Werkzeug, dadurch gekennzeichnet, daß das Spannelement (4) ständig auf dem Schaft (1) angeordnet ist, das erste Verriegelungselement (20) das Spannelement (4) in zwei axial beabstandeten Raststellungen am Schaft (1) lösbar verriegelt, das zweite Verriegelungselement (5) radial gleitend im Spannelement (4) geführt und mit dem radial inneren Ende (27) am Schaft (1) schwenkbar so gelagert ist, daß sein radial äußeres Ende zwecks Eingriff mit der Ausnehmung (34) des Werkzeugs (33) in einer der beiden Raststellungen aus dem Spannelement (4) vorsteht.

2. Spannvorrichtung nach Anspruch 1, dadurch gekennzeichnet, daß zwischen Schaft (1) und Spannelement (4) eine oder mehrere radial gelagerte Kugeln (20) angeordnet sind, die mit axial beabstandeten Ausnehmungen (13, 14) von Schaft oder Spannelement zusammenwirken.

0015433

3. Spannvorrichtung nach Anspruch 2, dadurch gekennzeichnet, daß die Ausnehmungen im Schaft (1) vorgesehen sind, vorzugsweise als Ringnuten (13, 14).

4. Spannvorrichtung nach Anspruch 3, dadurch gekennzeichnet, daß die Kugel jeweils in einer radialen Bohrung des Spannelements (4) angeordnet sind und radial außen gegen ein elastisch nachgebendes, die Bohrung umgebendes Ringelement (21) anliegen, das seinerseits in einer Ringausnehmung des Spannelements (4) angeordnet ist und das Ringelement (21) von einer dünnen Hülse (22) umgeben ist.

5. Spannvorrichtung nach einem der Ansprüche 1 - 4, dadurch gekennzeichnet, daß die Verriegelungselemente Stifte (5) sind, die in radialen Schlitzen (25) des Spannelements (4) angeordnet sind und die radial inneren Enden der Verriegelungsstifte (5) mit einer Ausnehmung (24) im Schaft (1) zusammenwirken.

6. Spannvorrichtung nach Anspruch 5, dadurch gekennzeichnet, daß die radial inneren Enden der Stifte (5) kugelförmig (27) sind.

7. Spannvorrichtung nach einem der Ansprüche 1 - 6, dadurch gekennzeichnet, daß der mit einer Bohrung zusammenwirkende Endabschnitt des Schaftes (1) von einem mit den Verriegelungselementen (5) zusammenwirkenden Endelement (6) gebildet wird.

0015433

8. Spannvorrichtung nach einem der Ansprüche 1 - 7, dadurch gekennzeichnet, daß zum Einspannen des Werkzeugs (33) das Spannelement (4) und Schaft (1) aufeinander zu bewegt werden und der Schaft (1) eine Schulter aufweist, die in der Spannstellung mit einem axialen Anschlag (29) des Spannelements (4) zusammenwirkt.

9. Spannvorrichtung nach Anspruch 7 und 8, dadurch gekennzeichnet, daß in der Trennstellung das Endelement (6) mit einem Anschlag (38) des Spannelements (4) zusammenwirkt.

10. Spannvorrichtung nach einem der Ansprüche 5 - 9, dadurch gekennzeichnet, daß die radialen Schlitze (25) axial nach außen offen sind und die Stirnseite des Spannelements (4) durch eine im Durchmesser kleinere Scheibe (8) abgedeckt wird, gegen deren Außenumfang sich die Stifte (5) bei der Verstellung von der Trenn- in die Spannstellung anlegen.

11. Spannvorrichtung nach einem der Ansprüche 5 - 10, dadurch gekennzeichnet, daß die Spannstifte (5) in der Trennstellung zur Schaftachse geneigt angeordnet sind und bei der Verstellung in die Spannstellung geringfügig über ihre Totpunktlage, in der sie in einer gemeinsamen radialen Ebene liegen, hinaus verstellt werden.

# FIG.1

# FIG.2

# FIG.3

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | AT - B - 345 445 (H. WEIGAND UND ANDERE)<br>+ Seite 5, Zeile 50 bis Seite 6, Zeile 9; Seite 6, Zeilen 23-34; Seite 7, Zeilen 4-16; Fig. 26,27,31,36,37 +<br>-- | 1-4,8 |
| A | US - A - 4 131 116 (PEVRICK ENG.)<br>+ Gesamt +<br>-- | |
| A | US - A - 3 702 611 (M. FISHBEIN)<br>+ Gesamt +<br>---- | |

**KLASSIFIKATION DER ANMELDUNG (Int.Cl. 3)**

A 61 B 17/00
A 61 B 17/16
A 61 F 1/00
B 23 Q 3/12

**RECHERCHIERTE SACHGEBIETE (Int. Cl. 3)**

A 61 B 17/00
A 61 F 1/00
B 23 B 29/00
B 23 B 31/00
B 23 Q 3/00
A 61 C 1/00
A 61 C 3/00

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

| X | Der vorliegende Recherchenbericht wurde für alle Patentansprüche erstellt. |
|---|---|

| Recherchenort | Abschlußdatum der Recherche | Prüfer |
|---|---|---|
| WIEN | 09-06-1980 | LUDWIG |

EPA form 1503.1  06.78